Europäisches Patentamt

European Patent Office

Office européen des brevets

(11) Publication number: **0 283 051**
**A1**

# EUROPEAN PATENT APPLICATION

(21) Application number: 88104493.7

(22) Date of filing: 21.03.88

(51) Int. Cl.⁴: **C12N 15/00** , C12P 17/00 , C12P 17/18

(30) Priority: 20.03.87 JP 66787/87
26.08.87 DE 3728506

(43) Date of publication of application:
21.09.88 Bulletin 88/38

(84) Designated Contracting States:
DE FR GB

(71) Applicant: LION CORPORATION
3-7, Honjo 1-chome
Sumida-ku Tokyo(JP)

(72) Inventor: Kamada, Hiroshi
2-1863, 110-302, Namiki Sakura-mura
Niihari-gun Ibaragi-ken(JP)
Inventor: Saga, Hitoshi
784-1, Shibusawa
Hadano-shi Kanagawa-ken(JP)

(74) Representative: Kuhnen, Wacker & Partner
Schneggstrasse 3-5 Postfach 1553
D-8050 Freising(DE)

(54) Method for preparing alkaloids.

(57) A method for preparing alkaloids, in particular tropane alkaloids, comprises the steps of transforming cells of a solanaceous plant by Ri plasmid present in Agrobacterium rhizogenes, culturing hairy roots resulting from the transformed cells in a medium and continuously recovering the alkaloids secreted by the hairy roots into the medium during the culturing the hairy roots. This method makes it possible to produce alkaloids from the hairy roots of solanaceous plants efficiently and thus is quite suitable for industrially producing useful alkaloids.

EP 0 283 051 A1

## Method for preparing alkaloids

Background of the Invention

(1) Field of the Invention

The present invention relates to an effective method for continuously preparing alkaloids, i.e., biogenic substances and medicinal substances biosynthesized by solanaceous plants, in particular tropane alkaloids, by culturing hairy roots of solanaceous plants.

(2) Prior Art

As industrial processes for preparing secondary metabolites of plants according to methods of culturing cells and tissues of plants, there is known a method in which Agrobacterium rhizogenes is inoculated into plants and the roots which appear are cultured. The method is based on the following: when A. rhizogenes is inoculated into plant stalks, leaves, roots and the like, roots, so-called hairy roots generate from the infected parts. It has been known that the roots generate in cases where a part of a gene of a huge plasmid (Ri plasmid) present in the A. rhizogenes is integrated into a gene of the plant, that growth of the roots is very fast, and that the amount of secondary metabolites thus produced is greater than that of ordinary roots. Accordingly, the method is a method utilizing the above knowledge, which comprises inoculating A. rhizogenes into plants producing useful substances in their roots, collecting the formed roots by cutting, culturing them in various apparatuses such as a tank, and crushing the grown roots to obtain the useful substances. This method has attracted attention as a method for preparing natural products and is considered feasible for practical use with some plants.

However, this method has disadvantages. First, the overall cost becomes high, since the target substances can not be obtained without crushing the cells and separating them from the cell components and second, the method can only be conducted by the batch method since it is difficult to continuously take out the growing plant organs.

Summary of the Invention

Under such circumstances, the inventors of the present invention previously developed a method which does not suffer from the foregoing drawbacks and comprises inoculating the aforementioned A. rhizogenes into specific plants, culturing the generated hairy roots in a culture medium and extracting alkaloids secreted by the hairy roots in the medium (see U.S. Serial No. 021392 filed on March 4, 1987). In addition, the inventors developed methods which make use of a liquid medium which contains specific elements in specific amounts or is free of such elements (see Japanese Patent Appln. Nos. 61-181532 and 61-181533).

However, when the hairy roots are cultured according to a batch method in which the culture is continued in the presence of alkaloids secreted into the medium, it is not possible to achieve a sufficiently high growing rate of the hairy roots and a large amount thereof secreted by the hairy roots into the medium.

Accordingly, it is a primary purpose of the present invention to provide a method for preparing alkaloids, which is suitable for use in effectively growing hairy roots obtained by transforming plant cells by Ri plasmid for the production of alkaloids.

It is another purpose of the present invention to provide a method for preparing alkaloids, which enables a higher growing rate of the hairy roots and high efficiency in the production of the alkaloids.

The present invention has been accomplished on the basis of the finding that alkaloids secreted in a medium by the hairy roots of solanaceous plants inhibit the growing of the roots and the production of alkaloids therein and, therefore, the aforementioned problems can effectively be solved by continuously removing and recovering the secreted alkaloids from the culture medium during culturing.

The above-described and other purposes of the present invention can effectively be accomplished by a method for preparing alkaloids which comprises transforming cells of a solanaceous plant by Ri plasmid present in A. rhizogenes, culturing the formed hairy roots in a medium and continuously recovering alkaloids secreted by the hairy roots into the medium during culturing of the hairy roots.

Description of the Preferred Embodiments

The method of the present invention is applicable to solanaceous plants and this limitation of the plants is very important in the present invention. More specifically, contrary to common belief, hairy roots of solanaceous plants can secrete alkaloids into a medium. Although any solanaceous plants can be used in the present invention, it is preferable to use a plant selected from the group consisting of Atropa sp., Datura sp., Hyoscyamus sp. and Scopolia sp..

Examples of A. rhizogenes usable to obtain the hairy roots of the above plants include A. rhizogenes 25818 (ATCC 25818), A. rhizogenes 15834 (ATCC 15834), A. rhizogenes 8196, A. rhizogenes A4 (ATCC 43057) and the like. There can also be used other bacteria, into which Ri plasmid or T-DNA forming a part of the plasmid is integrated, such as Escherichia coli.

According to the present invention, plants are treated with A. rhizogenes so that a part (T-DNA) of the Ri plasmid in the A. rhizogenes is integrated into genomic DNA of the plant cells (transformation).

Examples of methods for preparing hairy roots transformed by integrating Ri plasmid T-DNA into stalks, roots, leaves and the like of said solanaceous plants include the following methods (1) to (5) :

(1) Direct inoculation method applied to plants.

(2) Leaf disk transformation-regeneration method using pieces of leaf (R.B. Horsch et al., SCIENCE, 227, 1229 (1985)).

(3) Co-culture method utilizing protoplast of plants (Z.M. Wei et al., Plant Cell Rep., 5: 93-96 (1986)).

(4) Spheroplast method utilizing A. rhizogenes and protoplast of plants (R. Hain et al., Plant Cell Rep., 3, 60 (1984)).

(5) A method of direct injection of the Ri plasmid of A. rhizogenes or the T-DNA part of the plasmid into plant cells by microinjection or the like.

In the case where Ri plasmid is integrated into the genomic DNA of the plant cells by one of the methods (1) to (4), it is necessary to eliminate A. rhizogenes after the integration. The elimination of A. rhizogenes may be carried out by one of the following methods.

o    High temperature treatment (40°C).

o    Treatment with antibiotics.

o    Subculturing the root apex of the hairy roots in a short cycle.

It is preferable that the hairy roots obtained by the above methods can be cultured according to the following methods.

In the methods of this invention, there can be used conventional media which are employed in tissue-culture of plants. Such media comprise inorganic components and carbon sources as essential components and other components such as growth regulators, vitamins, amino acids as optional components.

Examples of the inorganic components include nitrogen, zinc, iron, copper, molybdenum, boron, phosphorous, cobalt, potassium, calcium, magnesium, sulfur, manganese, chlorine, sodium and iodine. These inorganic components are generally added to the media in the form of compounds and specific examples thereof are ammonium nitrate, ammonium dihydrogenphosphate, ammonium sulfate, ammonium chloride, sodium nitrate, calcium nitrate, potassium nitrate, zinc sulfate, ferrous sulfate, ferric sulfate, ferric monosodium ethylenediaminetetraacetate, copper sulfate, molybdic acid, sodium molybdate, boric acid, phosphoric acid, sodium dihydrogenphosphate, potassium dihydrogenphosphate, disodium hydrogenphosphate, trisodium phosphate, cobalt chloride, potassium chloride, calcium chloride, magnesium sulfate, sodium sulfate, manganese sulfate and potassium iodide.

On the other hand, examples of carbon sources include sucrose, other carbohydrates and their derivatives, organic acids such as fatty acids and primary alcohols such as ethanol.

Examples of growth regulators are auxins such as indoleacetic acid (IAA), naphthaleneacetic acid (NAA), p-chlorophenoxyisobutyric acid, 2,4-dichlorophenoxyacetic acid (2,4-D); and cytokinins such as kinetin, zeatin and dihydrozeatin.

There may be mentioned such vitamins as biotin, thiamine (vitamin $B_1$), pyridoxine (vitamin $B_6$), pantothenic acid, ascorbic acid (vitamin C), inositol and nicotinic acid.

As examples of amino acids, there may be named glycine, alanine, glutamine and cysteine.

In the methods of this invention, a variety of media containing the components listed above can be used. However, it is preferable to use liquid media. In this respect, the concentration of these components in the liquid media may vary widely. Generally, the liquid media can contain about 0.1 μM to about 1000 mM of inorganic components, about 1 g/l to about 120 g/l of carbon sources, about 0.01 μM to about 10 μM of growth regulators, about 0.1 mg/l to about 100 mg/l of vitamins and about 0.1 mg/l to about 100 mg/l of amino acids.

In the methods of the present invention, the amount of hairy roots to be initially inoculated into the

3

liquid medium can widely be varied. However, it is generally desirable that the hairy roots be inoculated into the medium in an amount of about 10 mg to about 1 g (weight of the fresh roots) per 50 ml of the liquid medium.

When culturing the hairy roots in accordance with the present invention, irradiation of the roots with light is not necessarily needed. On the contrary, culture in the dark is desirable for the biosynthesis of alkaloids. The culturing temperature preferably ranges from about 10°C to about 35°C, in particular, from about 23°C to about 28°C. This is because, if the temperature is less than about 10°C, the rate of proliferation of the hairy roots is low, while if it is more than about 35°C, the proliferation rate thereof also becomes low.

According to the methods of this invention, the alkaloids secreted by the hairy roots into the medium are continuously recovered while the culturing of the hairy roots is continued. The recovery of alkaloids can be effectively carried out accordingly to the following methods. In this connection, the operation of "continuous recovery" is defined to include recovery which is periodically effected at regular intervals.

When the culture of the hairy roots is effected according to the batch technique, the alkaloids secreted into the medium can be recovered by introducing a material capable of adsorbing alkaloids into the medium to continuously adsorb and recover the same. As such materials, there may be mentioned such adsorbents as silica gel, alumina, active carbon, Amberlite (available from ORGANO CO., LTD.) and SEPABEADS (available from MITSUBISHI CHEMICAL INDUSTRIES LTD.). During culturing of the hairy roots the medium is preferably stirred or shaken in order to assure the continuous adsorption of the alkaloids with the adsorbent.

In place of such adsorbents, it is also possible to use an organic solvent (or extracting medium) which is not compatible with the medium and can dissolve alkaloids. Examples thereof are liquid paraffin, heptamethylnonane and methyl myristate. In this case, it is also preferable that the medium be likewise stirred or shaken for the purpose of assuring the continuous removal of alkaloids secreted into the medium.

The adsorbents or the organic solvents are desirably used in an amount of 0.00001 to 10 times, preferably 0.001 to 1 time the weight of the medium.

Moreover, the recovery of the alkaloids can also be continuously effected according to a method comprising circulating the medium through an apparatus for recovering alkaloids disposed in the course of the circulation path and bringing it back to the culturing container. Examples of such apparatuses include those adapted for enclosing the foregoing adsorbent or the organic solvent in a container such as a column and passing the medium including the alkaloids therethrough.

The alkaloids can be continuously recovered by continuously removing a part of the used medium and continuously supplying fresh medium to the culturing container. The supply of the fresh medium may be carried out continuously or it is alternatively possible to intermittently replace a part or the whole of the used medium with fresh medium.

Thus, according to the methods of this invention, alkaloids can effectively be prepared using hairy roots of solanaceous plants. Therefore, the methods of the present invention is very suitable for industrial production of alkaloids.

The present invention will be further explained with respect to non-limitative examples.

Example 1

Seeds of <u>Datura</u> <u>innoxia</u> Mill. were sterilized by a sterilization agent such as sodium hypochlorite solution and thereafter, the seeds were placed on 3% sucrose containing Murashige & Skoog (referred to as MS-3; shown in Table-1) solid medium to obtain seedings. <u>A.</u> <u>rhizogenese</u> (ATCC 15834) harboring Ri plasmid was inoculated into the thus obtained stalks or leaves or other parts of the germ-free plants.

Two to five weeks later, hairy roots appearing at the infected parts were collected by cutting, transferred on MS-3 solid medium containing 1 g/l of carbenicillin, and after one or two weeks, were further transferred on fresh MS-3 solid medium containing the same components as described above. The above procedure was repeated two or three times to obtain sterilized hairy roots.

On the other hand, 50 ml of the MS-3 liquid medium was charged into a 100 ml of Erlenmeyer flask and then sterilized at a temperature of 120°C for 15 minutes. The above-described hairy roots (100 mg; weight of fresh roots) were transferred into the liquid medium and cultured by the shaking culture technique (100 rev/min.; 30 mm in amplitude) at 25°C, in the dark for 15 days. On the 16th day, the hairy roots were transferred to containers each of which contained 50 ml of fresh MS-3 liquid medium including Amberlite XAD-2, 4 or 7 or SEPABEADS HP-20 in the amount shown in Table-2. The liquid medium had been sterilized under the same conditions as described above. Then, the culturing of the roots was effected for an additional 15 days.

After the culturing, the hairy roots of D. innoxia M. and the adsorbents were collected by filtration. The hairy roots were weighed, and subjected to freeze-drying. The dried hairy roots were also weighed and ground into a powder using a mortar and a pestle. The powder was then extracted with a mixture of chloroform, methanol and ammonia (ratio = 15:5:1). And then the mixture was filtrated to obtain an extract. Thus obtained extract was then subjected to extraction by chloroform at pH 2 (adjusted with sulfuric acid) to obtain an aqueous phase, which was further extracted with chloroform at pH 10 (adjusted with ammonia). The thus obtained chloroform phase was dehydrated by using sodium sulfate anhydride and then evaporated to dryness under a reduced pressure to obtain alkaloid fraction.

The liquid medium was weighed and thereafter the pH of the medium was adjusted to 2 with sulfuric acid and the medium was subjected to the same extraction method to isolate alkaloid fraction.

Amberlite XAD-2, 4, 7 and SEPABEADS HP-20 were weighed and then extracted with chloroform. The extract was evaporated to dryness under a reduced pressure and the residue was extracted in the same manner as described above in connection with the hairy roots, whereby an alkaloid fraction was obtained.

Quantitative analysis of atropine and scopolamine present in the alkaloid fractions was effected by gas chromatography. The column used was OV-17 (2 m $\times$ 3 mm), the temperature thereof was 235°C and the carrier gas used was nitrogen at a flow rate of 50 ml/min. A detector FID was used.

The total amount of atropine or scopolamine produced per flask was determined by summing up the amounts thereof obtained from the three alkaloid fractions.

The results thus obtained and the amount of Amberlite XAD-2, 4 and 7 and SEPABEADS HP-20 added to the medium are listed in Table-2. Each result in Table-2 is the average of three measurements. In Table-2, AT and SC mean atropine and scopolamine, respectively (those in the following Tables being expressed in the same manner).

Table-1: Components of MS-3 medium

| Classification | Component | Concentration (unit) | |
|---|---|---|---|
| Inorganic components | $NO_3^-$ | 39.4 | (mM) |
| | $PO_4^{3-}$ | 1.25 | (mM) |
| | $SO_4^{2-}$ | 1.5 | (mM) |
| | $Cl^-$ | 6.0 | (mM) |
| | $K^+$ | 20.0 | (mM) |
| | $Ca^{2+}$ | 3.0 | (mM) |
| | $Mg^{2+}$ | 1.5 | (mM) |
| | $NH_4^+$ | 20.6 | (mM) |
| | $Mn^{2+}$ | 100 | ($\mu$M) |
| | $Zn^{2+}$ | 30 | ($\mu$M) |
| | $BO_3^{3-}$ | 100 | ($\mu$M) |
| | $I^-$ | 5 | ($\mu$M) |
| | $Cu^{2+}$ | 0.1 | ($\mu$M) |
| | $Na^+$ | 200 | ($\mu$M) |
| | $Co^{2+}$ | 0.1 | ($\mu$M) |
| | $Mo^{6+}$ | 1 | ($\mu$M) |
| | $Fe^{2+}$ | 100 | ($\mu$M) |
| Vitamins | myo-inositol | 555 | ($\mu$M) |
| | nicotinic acid | 4.06 | ($\mu$M) |
| | pyridoxine-HCl | 2.43 | ($\mu$M) |
| | thiamine-HCl | 0.3 | ($\mu$M) |
| Amino acid | glycine | 26.6 | ($\mu$M) |
| Carbohydrate | sucrose | 87.7 | (mM) |

Table 2

| | Adsorbent | | Amount of alkaloid in the hairy roots (mg) | | Amount of alkaloid in the medium (mg) | | Amount of alkaloid in the adsorbent (mg) | | Total amount of alkaloid (mg) | |
|---|---|---|---|---|---|---|---|---|---|---|
| | Kind | Amount | AT | SC | AT | SC | AT | SC | AT | SC |
| Present Invention | Amberlite XAD-2 | 0.3 g | 3.65 | 1.22 | 0.96 | 0.35 | 1.40 | 0.50 | 6.01 | 2.07 |
| | | 0.6 g | 1.65 | 0.47 | 0.05 | 0.01 | 5.58 | 1.25 | 7.28 | 1.73 |
| | Amberlite XAD-4 | 0.3 g | 1.59 | 0.32 | 0.68 | 0.14 | 5.08 | 1.16 | 7.35 | 1.62 |
| | | 0.6 g | 1.41 | 0.32 | 0.03 | 0.00 | 7.90 | 1.58 | 9.34 | 1.90 |
| | Amberlite XAD-7 | 0.3 g | 2.31 | 0.71 | 0.31 | 0.10 | 0.32 | 0.13 | 2.94 | 0.94 |
| | | 0.6 g | 2.15 | 0.64 | 0.54 | 0.13 | 1.51 | 0.50 | 4.20 | 1.27 |
| | SEPABEADS HP-20 | 0.3 g | 1.70 | 0.23 | 0.70 | 0.17 | 4.52 | 1.26 | 6.92 | 1.66 |
| | | 0.6 g | 1.53 | 0.32 | 0.04 | 0.01 | 6.80 | 1.50 | 8.37 | 1.83 |
| Comparative Example | Not added | — | 1.64 | 0.48 | 0.06 | 0.13 | — | — | 1.70 | 0.61 |

As seen from the results summarized in Table-2, a large amount of alkaloids can be produced according to the method of this invention, compared with the Comparative Example in which the alkaloids secreted into the medium were not recovered during the culturing.

Example 2

Hairy roots of Datura innoxia M. and a crossing of Duboisia myoporoides $\times$ D. leichardtii were cultured and the resultant alkaloids were quantitatively analyzed in the same manner as described in Example 1 except that the hairy roots were, in turn, transferred to fresh MS-3 liquid media having the same composition at the intervals shown in Tables-3 and 4, without using adsorbents.

The intervals at which the medium was replaced by fresh medium and the results obtained are summarized in Tables-3 and 4.

Table-3

<u>D</u>. <u>innoxia</u> M.

| | Interval of medium-replacement (days) | Amount of alkaloid in the hairy roots (mg) | | Amount of alkaloid in the whole medium (mg) | | Total amount of alkaloid (mg) | |
|---|---|---|---|---|---|---|---|
| | | AT | SC | AT | SC | AT | SC |
| Present Invention | 3 | 2.27 | 0.56 | 9.87 | 2.40 | 12.1 | 2.96 |
| | 5 | 2.55 | 0.57 | 6.13 | 1.78 | 8.68 | 2.35 |
| Comparative Example | not replaced | 1.64 | 0.48 | 0.06 | 0.13 | 1.70 | 0.61 |

0 283 051

## Table-4

Duboisia myoporoides X D. leichardtii

|  | Interval of medium-replacement (days) | Amount of alkaloid in the hairy roots (mg) | | Amount of alkaloid in the whole medium (mg) | | Total amount of alkaloid (mg) | |
|---|---|---|---|---|---|---|---|
|  |  | AT | SC | AT | SC | AT | SC |
| Present Invention | 3 | 0.44 | 0.33 | 0.46 | 1.08 | 0.90 | 1.41 |
|  | 5 | 0.20 | 0.14 | 0.49 | 1.07 | 0.69 | 1.21 |
| Comparative Example | not replaced | 0.13 | 0.14 | 0.02 | 0.16 | 0.15 | 0.30 |

0 283 051

As is obvious from Tables-3 and 4, the method of the present invention makes it possible to prepare a large amount of alkaloids, compared with the Comparative Example in which the medium was not replaced at all during the culturing.

Example 3

An air-lift type plant culturing device of 2 liters volume (available from SHIBATA-HARIO GLASS CO., LTD.) was charged with 1 ℓ of MS-3 liquid medium. The device was fitted with a circulator for continuously circulating the medium at the rate of 40 ml/hr. during culturing. The circulator was connected thereto through pump, glass tubes and silicone tubes. In the flow path of the medium, there was fitted a glass column packed with 20 g of Amberlite XAD-4, having a diameter of 35 mm and a length of 150 mm so that the circulating medium passed therethrough. The devices were sterilized by holding them at 120°C for 15 minutes.

The hairy roots of D. innoxia M. and Atropa belladonna L. prepared in the same manner as used in Example 1 (about 1 g; weight of fresh roots) were inoculated into the liquid medium and were cultured in the dark at 25°C for 30 days. Separately, the same procedures were repeated except for using an apparatus in which the glass column was not packed with Amberlite XAD-4 (Comparative Example).

After culturing, the amounts of alkaloids were determined in the same manner as in Example 1.

The results observed are listed in Tables-5 and 6. As seen from these results, a large amount of alkaloids can be obtained by the method of this invention, compared with the Comparative Example in which the alkaloids secreted into the medium during the culturing were not removed or recovered.

11

Table-5

<u>D. innoxia</u> M.

| | Amount of alkaloid in the hairy roots (mg) | | Amount of alkaloid in the medium (mg) | | Amount of alkaloid in the adsorbent (mg) | | Total amount of alkaloid (mg) | |
|---|---|---|---|---|---|---|---|---|
| | AT | SC | AT | SC | AT | SC | AT | SC |
| Present Invention | 17.62 | 2.09 | 3.40 | 0.31 | 104.8 | 14.85 | 125.8 | 17.25 |
| Comparative Example | 22.83 | 7.69 | 2.91 | 1.47 | — | — | 25.74 | 9.16 |

0 283 051

Table-6

<u>A</u>. <u>belladonna</u> L.

| | Amount of alkaloid in the hairy roots (mg) | | Amount of alkaloid in the medium (mg) | | Amount of alkaloid in the adsorbent (mg) | | Total amount of alkaloid (mg) | |
|---|---|---|---|---|---|---|---|---|
| | AT | SC | AT | SC | AT | SC | AT | SC |
| Present Invention | 8.01 | 0.17 | 1.50 | 0.02 | 36.0 | 0.88 | 45.5 | 1.07 |
| Comparative Example | 18.3 | 0.46 | 0.64 | 0.04 | – | – | 18.9 | 0.50 |

Example 4

An air-lift type plant culturing device of 2 ℓ volume was charged with 1 ℓ of MS-3 liquid medium and was fitted with a device for continuously replacing a part of the used medium for fresh medium at a flow rate of 4 ml/hr., through pumps, glass tubes and silicone tubes. In other words, 3 ℓ of the fresh MS-3 liquid medium stored in a preserve jar was injected into the culturing device through the first pump at a flow rate of 4 ml/hr. while the medium containing alkaloids was continuously discharged from the culturing device through the second pump at the same flow rate and was stored in a separate preserve jar. The devices were sterilized by holding at 120° for 15 minutse.

About 1 g (weight of fresh roots) of hairy roots of Hyoscyamus muticus L. prepared in the same manner as explained in Example 1 were inoculated into the medium and were cultured, in the dark, at 25°C for 30 days.

After culturing, the amount of alkaloids produced was determined in the same manner as described in Example 1. The results obtained are summarized in Table-7 given below.

Table-7

H. muticus L.

|  | Amount of alkaloid in the hairy roots (mg) | | Amount of alkaloid in the medium (mg) | | Total amount of alkaloid (mg) | |
|---|---|---|---|---|---|---|
|  | AT | SC | AT | SC | AT | SC |
| Present Invention | 1.15 | 2.56 | 4.46 | 12.4 | 5.61 | 15.0 |
| Comparative Example | 1.63 | 4.82 | 0.37 | 1.12 | 2.00 | 5.94 |

As is obvious from the results listed in Table-7, a large amount of alkaloids is produced according to the method of this invention, compared with the Comparative Example in which the alkaloid secreted was not recovered from the medium.

Example 5

An air-lift type plant culturing device of 3 ℓ volume was charged with 3 ℓ of liquid medium, namely, Woody Plant Medium containing 3% sucrose (referred to as WP-3; shown in Table-9), and was fitted with a circulator for continuously circulating the medium at a flow rate of 40 ml/hr. during culturing, the circulator being connected to the culturing device through pumps, glass tubes and silicone tubes. In addition, a glass column packed with 20 g of Amberlite XAD-4, having a diameter of 17 mm and a length of 300 mm, was fitted in the flow path of the medium, so that the circulating medium passed therethrough. These devices were sterilized at 120°C for 15 minutes.

About 1 g (weight of fresh hairy roots) of the hairy roots of Hyoscyamus albus L. prepared in the same manner as described in Example 1 were inoculated into the medium and were cultured, in the dark, at 25°C for 23 days followed by additional culturing, likewise in the dark, at 25°C for 21 days while operating the pump to circulate the medium.

Separately, the same procedures were repeated except that Amberlite XAD-4 was removed from the glass column fitted to the plant culturing device (Comparative Example).

After the completion of culturing, the amounts of alkaloids were determined in the same manner as explained in Example 1.

The results obtained are given in Table-8. As is obvious from these results, a large amount of alkaloids is produced according to the method of this invention, compared with the Comparative Example in which the alkaloids secreted into the medium were not recovered during culturing.

Table-8

<u>H</u>. <u>albus</u> L.

| | Amount of alkaloid in the hairy roots (mg) | | Amount of alkaloid in the medium (mg) | | Amount of alkaloid in the adsorbent (mg) | | Total amount of alkaloid (mg) | |
|---|---|---|---|---|---|---|---|---|
| | AT | SC | AT | SC | AT | SC | AT | SC |
| Present Invention | 17.7 | 56.9 | 2.13 | 8.37 | 16.5 | 438.0 | 36.3 | 503.3 |
| Comparative Example | 91.0 | 78.8 | 8.34 | 12.1 | − | − | 99.3 | 90.9 |

0 283 051

Table-9: COMPONENTS OF WP-3 MEDIUM

| Classification | Component | Concentration (unit) | |
|---|---|---|---|
| Inorganic components | $NO_3^-$ | 9.7 | (mM) |
| | $PO_4^{3-}$ | 1.25 | (mM) |
| | $SO_4^{2-}$ | 7.34 | (mM) |
| | $Cl^-$ | 1.3 | (mM) |
| | $K^+$ | 12.61 | (mM) |
| | $Ca^{2+}$ | 3 | (mM) |
| | $Mg^{2+}$ | 1.5 | (mM) |
| | $NH_4^+$ | 5 | (mM) |
| | $Mn^{2+}$ | 130 | ($\mu$M) |
| | $Zn^{2+}$ | 30 | ($\mu$M) |
| | $BO_3^{3-}$ | 100 | ($\mu$M) |
| | $Cu^{2+}$ | 1 | ($\mu$M) |
| | $Na^+$ | 2 | ($\mu$M) |
| | $Mo^{6+}$ | 1 | ($\mu$M) |
| | $Fe^{3+}$ | 100 | ($\mu$M) |
| Vitamins | myo-inositol | 555 | ($\mu$M) |
| | nicotinic acid | 4.06 | ($\mu$M) |
| | pyridoxine-HCl | 2.43 | ($\mu$M) |
| | thiamine-HCl | 3 | ($\mu$M) |
| Amino acid | glycine | 26.6 | ($\mu$M) |
| Carbohydrate | sucrose | 87.7 | (mM) |

Claims

1. A method for preparing alkaloids comprising transforming cells of a solanaceous plant by Ri plasmid present in Agrobacterium rhizogenes, culturing hairy roots resulting from the transformed cells in a medium and continuously recovering alkaloids secreted by the hairy roots into the medium during the culturing of the hairy roots.

2. A method as set forth in claim 1 wherein the alkaloids secreted into the medium are continuously recovered by adsorbing them with an adsorbent.

3. A method as set forth in claim 1 wherein the alkaloids secreted by the hairy roots are continuously recovered by dissolving them into an organic solvent which is not compatible with the medium and is capable of dissolving the alkaloids therein.

4. A method as set forth in claim 3 wherein the organic solvent is a member selected from the group consisting of liquid paraffin, heptamethylnonane, and methyl myristate.

5. A method as set forth in claim 1 wherein the medium is a liquid medium and the recovery of the alkaloids secreted into the medium is effected by continuously discharging a part of the medium from a culturing device while continuously supplying fresh medium to the device.

6. A method as set forth in claim 1 wherein the alkaloids secreted into the liquid medium is continuously recovered by intermittently replacing a part or the whole of the medium with fresh medium.

7. A method as set forth in claim 1 wherein the alkaloids secreted into the liquid medium are continuously recovered by circulating the medium through a device for recovering the alkaloids, disposed in the flow path of the medium.

8. A method as set forth in claim 7 wherein the device for recovering the alkaloids is a column packed with an adsorbent or an organic solvent which is not compatible with the medium but is capable of dissolving the alkaloids.

9. A method as set forth in claim 1 wherein the solanaceous plant is a member selected from the group consisting of Atropa sp., Datura sp., Hyoscyamus sp. and Scopolia sp.

10. A method as set forth in claim 1 wherein the alkaloids are tropane alkaloids.

11. A method as set forth in claim 10 wherein the tropane alkaloids are atropine and scopolamine.

| DOCUMENTS CONSIDERED TO BE RELEVANT | | | EP 88104493.7 |
|---|---|---|---|
| Category | Citation of document with indication, where appropriate, of relevant passages | Relevant to claim | CLASSIFICATION OF THE APPLICATION (Int. Cl.4) |
| A | WO - A1 - 84/03 298 (RESEARCH AND DEVELOPMENT INSTITUTE, INC.) <br> * Pages 1-3; claims 1,9,10 * <br> -- | 1 | C 12 N 15/00 <br><br> C 12 P 17/00 <br><br> C 12 P 17/18 |
| A | NATURE, vol. 295, no. 5848, February 4, 1982, New York, London <br> M.-D.CHILTON et al. "Agrobacterium rhizogenes inserts T-DNA into the genomes of the host plant root cells" <br> pages 432-434 <br> * Totality * <br> -- | 1 | |
| A | PROCEEDINGS OF THE NATIONAL ACADEMY OF SCIENCES OF THE UNITED STATES OF AMERICA, vol. 79, no. 10, May 1982, Baltimore, USA <br> F.F.WHITE et al. "Tumor induction by Agrobacterium rhizogenes involves the transfer of plasmid DNA to the plant genome" <br> pages 3193-3197 <br> * Totality * <br> ---- | 1 | TECHNICAL FIELDS SEARCHED (Int. Cl.4) <br><br> C 12 N <br> C 12 P |

The present search report has been drawn up for all claims

| Place of search | Date of completion of the search | Examiner |
|---|---|---|
| VIENNA | 26-05-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82